# EUROPÄISCHE PATENTSCHRIFT

(11) **EP 4 362 864 B1**
(45) Veröffentlichungstag und Bekanntmachung des Hinweises auf die Patenterteilung: **07.05.2025**
(21) Anmeldenummer: 22740805.1
(22) Anmeldetag: 27.06.2022
(51) Int. Cl.: A61F 5/02

(54) **THORAX-ORTHESE**
THORACIC ORTHOSIS
ORTHÈSE THORACIQUE

(30) Priorität: 02.07.2021 DE 102021117132
(43) Veröffentlichungstag der Anmeldung: 08.05.2024
(73) Patentinhaber: AX-Lightness Composites GmbH, 37115 Duderstadt (DE)
(72) Erfinder: NOLTE, Bernd, 37115 Duderstadt (DE); LOTZ, Klaus, 35614 Aßlar (DE)
(74) Vertreter: Schneider, Peter Christian
(86) Internationale Anmeldenummer: PCT/EP2022/067617
(87) Internationale Veröffentlichungsnummer: WO 2023/274983

(56) Entgegenhaltungen:
- WO-A1-2020/186209
- CN-A- 107 898 544
- DE-A1- 102015 107 582
- DE-A1- 102016 201 270
- DE-A1- 4 117 768
- US-A- 4 648 390
- US-A1- 2008 045 873
- US-A1- 2013 261 521
- US-A1- 2021 077 336

## Beschreibung

### Gebiet der Erfindung

Die Erfindung bezieht sich auf eine Thorax-Orthese zur bedarfsweisen Entlastung der Wirbelsäule eines menschlichen Nutzers,
umfassend
- eine einstückig aus einem blattartigen, in ihrer Längserstreckungsrichtung stauchfesten Carbon-Material aufgebaute Rückenschiene, die im Gebrauchsfall mit einer ventralen Flachseite im Bereich der Brust- und zumindest der oberen Lendenwirbel des Nutzers an den der Wirbelsäule des Nutzers beidseitig benachbarten Rückenstreckermuskeln anliegt, sowie
- ein mit der Rückenschiene verbundenes Schulterträgersystem mit zwei im Gebrauchsfall die Schultern des Nutzers rucksackartig umgreifenden Trägerelementen.

### Stand der Technik

Eine derartige Thorax-Orthese ist bekannt aus der DE 10 2016 201 270 A1.

In vielen Situationen des täglichen Lebens ist die menschliche Wirbelsäule erheblichen Belastungen ausgesetzt. Dies beispielsweise beim Heben und Tragen schwerer Lasten vor dem Körper, wie beispielsweise beim Transport von Ziegeln, Zement oder Ähnlichem im Baugewerbe. Aber ebenso beim Transport von rucksackartig geschulterten Lasten, wie beispielsweise Sauerstoffflaschen bei Rettungseinsätzen der Feuerwehr. Auch in vielen Sportarten, beispielsweise dem Reitsport, insbesondere dem Springreitsport, ist die menschliche Wirbelsäule temporär erheblichen Belastungen ausgesetzt. Bei optimalem Bewegungsablauf handelt es sich bei all diesen Belastungen im Wesentlichen um in Längserstreckungsrichtung der Wirbelsäule, d. h. cranial-kaudal gerichtete Stauchbelastungen, die teilweise von der natürlichen S-Krümmung der Wirbelsäule in der medialen Sagittalebene abgefangen werden, wobei die externen Kräfte über die Wirbelsäule in die benachbarte Muskulatur abgeleitet und dort letztendlich dissipiert werden. Häufig lässt sich die jeweilige Bewegung jedoch nicht optimal koordinieren, sodass auch Biege-, Torsions- und Scherbewegungen auftreten können. Übermäßig hohe oder übermäßig lange Belastungen können nachteilige gesundheitliche Folgen haben. Dies umso mehr, je stärker seitlich die jeweiligen Kräfte angreifen und entsprechend schlechter von der natürlichen S-Krümmung der Wirbelsäule abgefangen werden können. Zum Überlastungsschutz der Wirbelsäule sind daher etliche Orthesen bekannt, denen die Aufgabe gemeinsam ist, die extern einwirkende Kräfte von der Wirbelsäule weg auf weniger empfindliche skelettale Strukturen des Körpers abzuleiten. Die Bezeichnung Orthese wird hier als ein Sammelbegriff für körpernahe Hilfsmittel verwendet, die eine Balance zwischen Beweglichkeit und Stabilität des Körpers gewährleisten sollen. Sie kommen nicht nur nach Verletzungen oder bei Fehlstellungen von Gliedmaßen, d. h. bei medizinisch-therapeutischer Indikation zum Einsatz, sondern auch - und im Rahmen der vorliegenden Erfindung insbesondere - ohne medizinische Indikation oder Zielsetzung zur rein vorbeugenden und unterstützenden Stabilisierung beanspruchter Körperbereiche.

Aus der DE 10 2011 076 843 B4ist eine Orthese mit einer aus einer Vielzahl separater Segmente aufgebauten Rückenschiene bekannt. Die einzelnen Segmente sind mit den jeweiligen Nachbarsegmenten über Federn verbunden. Zudem ist ein alle Segmente in Längserstreckung durchsetzendes Zugelement vorgesehen. Mittels eines Gurtsystems, bestehend aus zwei oberen Schultergurten und einem unteren Beckengurt lässt sich die Rückenschiene derart am Rücken des Nutzers fixieren, dass sich ihre einzelnen Segmente entlang der Wirbelsäule an den Rücken des Nutzers anschmiegen, wobei sie zentral auf der Wirbelsäule und lateral auf den der Wirbelsäule benachbarten Rückenstreckermuskeln aufliegen. Im deaktivierten Normalzustand erlauben es sie Federelemente zwischen den einzelnen Segmenten, dass die Rückenschiene den Bewegungen des Nutzers, insbesondere seiner Wirbelsäule, ohne nennenswerte Gegenkraft folgt. Zur Aktivierung einer Schutzfunktion kann mittels eines Schalters das sämtliche Segmente durchlaufende Zugelement angespannt werden, sodass die Segmente zu einem im Wesentlichen steifen Stab zusammengepresst werden, der sich zwischen dem Beckengurt und den Schultergurten abstützt. Auf die Schultern - insbesondere über die Arme - einwirkende Kräfte, wie sie etwa beim Aufheben schwerer Lasten auftreten, werden daher über die nun versteifte Rückenschiene an der Wirbelsäule vorbei auf den Beckengurt und somit auf das Becken des Nutzers abgeleitet. Um dem Benutzer auch im aktivierten Zustand eine gewisse, wenn auch stark eingeschränkte Bewegungsfreiheit zu belassen, sind die einzelnen Elemente aus hartelastischem Kunststoff gefertigt, was eine stark beschränkte Bewegung gegen erhebliche Gegenkräfte erlaubt.

Die bekannte Orthese muss in mehrerer Hinsicht als nachteilig angesehen werden. Zunächst bedarf es jeweils der bewussten Aktivierung bzw. Deaktivierung der Schutzfunktion vor bzw. nach dem Auftreten einer Belastung. Dies ist für den Nutzer umständlich und lästig und in allen Fällen, in denen Belastungen nicht mit deutlichem zeitlichen Vorlauf planbar sind, wie beispielsweise bei Rettungseinsätzen oder im Sport, schlicht untauglich. Sodann ist der beschriebene Mechanismus kompliziert und bauraumintensiv, sodass die bekannte Orthese unter bzw. über der Kleidung deutlich aufträgt. Zudem ist sie in Kombination mit auf dem Rücken getragenen Lasten, beispielsweise Sauerstoffflaschen, kaum einsetzbar, da das Flaschengewicht die dicke Rückenschiene schmerzhaft auf die Wirbelsäule des Nutzers drückt. Und schließlich besteht die Wirbelsäulen-Entlastung der bekannten Orthese lediglich in einer Verlagerung der Last von einer skelettalen Struktur, nämlich der Wirbelsäule, auf eine andere skelettale Struktur, nämlich das Becken, welches nur vermeintlich weniger empfindlich ist.

Eine ähnliche Orthese ist bekannt aus der WO 2013/156394 A1, wobei ein sehr komplexes System aus Zugseilen und Schiebern für eine sehr individuelle temporäre Anpassbarkeit der Orthese an die jeweilige Belastungssituation sorgt.

Aus der DE 20 2010 006 731 U1 ist eine Osteoporose-Orthese mit einer Pelotte in Form eines geschlossenen Rahmens aus einem Carbon-Werkstoff bekannt, die sich in einer Tasche befindet, welche mittels eines Gurtsystems an den Schultern sowie an Becken und Bauch des Trägers fixiert ist.

Auch die EP 1 902 691 B1 offenbart eine Osteoporose-Orthese mit einer Pelotte, die sich in einer Tasche befindet, welche mittels eines Gurtsystems an den Schultern sowie an Becken und Bauch des Trägers fixiert ist. Die Pelotte ist dabei in unterschiedlichen Vertikalbereichen aus unterschiedlichen Materialien gefertigt, um dort unterschiedliche Elastizitäten zu realisieren.

Eine besondere Pelottenform ist aus der DE 41 17 768 A1 bekannt, die Dornfortsätze der Wirbel kontaktfrei überspannt.

Gemeinsam ist sämtlichen Osteoporose-Orthesen, dass der durch die degenerative Erkrankung geschwächte Körper des Patienten mittels Gurten möglichst eng an einen stabilen Träger geschnallt werden soll. Die Bewegungsfreiheit des Patienten ist dabei von nachgeordneter Bedeutung. So auch bei der eingangs genannten, gattungsbildenden DE 10 2016 201 270 A1. Hier ist allerdings eine Orthese mit einstückiger, starrer Rückenschiene aus einem Carbon-Material beschrieben, die mittels eines breiten Beckengurtes und eines komplexen Schulter- und Rippengurtsystems Bewegungen des Patienten besser zulassen soll, indem sich das Gurtsystem - nicht etwa die starre Rückenschiene - aufgrund ihrer besonderen Gurtführung und -verschnallung den Bewegungen in beschränktem Umfang folgen kann.

Die WO 2020/186209 A1 offenbart eine mittels Schulter- und Beckengurten an den Körper des Benutzers geschnallte Rückenschiene, die nicht flach, sondern als dreidimensionale Körper ausgebildet ist, der sich ventral der S-Form der Wirbelsäule anschmiegt und dorsal eine abgeflachte mechanische Schnittstelle bildet, die die Kopplung mit zu tragenden Lasten vereinfacht.

Die US 2008/0045873 A1 offenbart eine aus vielen Einzelteilen aufgebaute, dick gepolsterte Thorax-Orthese, die durch Variation der Verknüpfungen der Einzelteile eine präzise Einstellung der mit ihr erzeugten Bewegungseinschränkung bzw. Stützwirkung für den Patienten erlaubt, um mit einer einzigen Orthese einen gesamten Genesungsverlauf nach einer Rückenverletzung abdecken zu können.

Auch die DE 10 2015 107 582 A1 offenbart eine ähnliche modulare Orthese, die allerdings auf Schultergurte verzichtet und die selbst gar nicht am Rücken des Patienten anliegende Rückenschiene mittels eines an der Brustmuskulatur abgestützten Brustbügels und eines Beckengurtes am Patientenkörper fixiert.

Aus der US 2013/0261521 A1 ist eine individuell an die Körpergröße des Benutzers anpassbare Tragehilfe bekannt, die aus einem steifen, das Becken des Benutzers umgreifenden, unteren Teil und einem ebenfalls steifen, an den Schultern und dem Rücken des Benutzers anliegenden, oberen Teil besteht, wobei beide Teile in vertikaler Richtung zueinander verschiebbar und in diversen Verschiebestellungen aneinander fixierbar sind.

Aus der US 2021/0077336 A1 ist eine Traghilfe bekannt, die die mechanische Belastung des Körpers beim Tragen von schweren Schutzschürzen, z.B. Bleischürzen für Röntgenpersonal, reduzieren soll. Hierzu wird dem Benutzer mittels eines Bauchgurtes eine stuhllehnenartige Vorrichtung aus einem textilbespannten Rahmen ins Kreuz geschnallt und ggf. mit zusätzlichen Schultergurten fixiert. Bei im Wesentlichen stehenden Tätigkeiten mag der damit verbundene Diskomfort für den Benutzer erträglich sein; für bewegungsträchtige Arbeiten oder gar für den Sport ist eine solche Vorrichtung untauglich.

Die US 4 648 390 A offenbart einen weitgehend steifen Schulter- und Halsring als Trägerbasis für unterschiedliche ankoppelbare und auf den Einzelfall zugeschnittene orthopädische Hilfsvorrichtungen.

Die CN 107898544 A offenbart einen um den Oberkörper eines Benutzers klappbaren Käfig, der als Rucksack und als Orthese dienen soll.

### Aufgabenstellung

Es ist die Aufgabe der vorliegenden Erfindung, eine variabel einsetzbare, für den Nutzer angenehmer zu tragende und der Gesundheit insgesamt zuträglichere Orthese zur Wirbelsäulen-Entlastung zu Verfügung zu stellen.

### Darlegung der Erfindung

Diese Aufgabe wird in Verbindung mit den Merkmalen des Oberbegriffs von Anspruch 1 dadurch gelöst, dass das Carbon-Material der Rückenschiene sagittal biegeelastisch ist und jedes Trägerelement des Schulterträgersystems aus einem blattartigen Carbon-Material aufgebaut ist und im Gebrauchsfall einen die zugeordnete Schulter des Nutzers umgreifenden Ring bildet, welcher, ausgehend vom kranialen Ende der Rückenschiene, flach an Schulter, Schlüsselbein und Brustmuskel des Nutzer anliegend parallel zu dessen Rippenbögen zur Rückenschiene zurückgeführt und im unteren Bereich deren oberen Drittels und/oder oberen Bereich ihres mittleren Drittels mit ihr verbunden ist.

Bevorzugte Ausführungsformen sind Gegenstand der abhängigen Ansprüche.

Zunächst folgende Anmerkungen zum Verständnis der hier verwendeten Begrifflichkeiten: Der Fachmann wird verstehen, dass die Vorteile der erfindungsgemäßen Orthese nur in ihrer Wechselwirkung mit dem Körper des Nutzers zur Geltung kommen können. Die räumlich-körperlichen Merkmale der erfindungsgemäßen Orthese lassen sich daher nur in Relation zum Körper des Nutzers beschreiben, was vorliegend mit dem Hinweis "im Gebrauchsfall" zum Ausdruck gebracht wird, wobei es selbstverständlich sein dürfte, dass der in Bezug genommene Körper des Nutzers nicht selbst Teil der Erfindung ist. Begriffe wie "ventral", "dorsal", "zentral", "lateral", "oben" bzw. "kranial", "unten" bzw. "kaudal" sind daher stets in diesem Sinne, d.h. in Relation zum aufrecht stehenden Nutzer-Körper bei bestimmungsgemäßem Gebrauch der erfindungsgemäßen Orthese zu verstehen. Vergleichbares gilt für Angaben von Ebenen wie "sagittal" oder "frontal", wobei mit einer derart spezifizierten Bewegung, Elastizität oder einem derart spezifizierten Verlauf stets die Bewegung, Elastizität bzw. der Verlauf in einer solchen Ebene gemeint ist.

Unter anderem erfindungswesentlich ist die besondere Wahl des Materials der Orthese und insbesondere ihrer Rückenschiene. Mit dem Begriff "Carbon-Material" ist hier ein kohlenstofffaserverstärkter Kunststoff gemeint, nämlich in eine duroplastische Matrix eingebettete, gerichtete Kohlenstofffasern. Insbesondere handelt es sich um gewebte, gelegte oder geflochtene Kohlenstofffasermatten, die in besagte duroplastische Matrix eingebettet sind. Wie dem Fachmann bekannt ist, ist es eine besondere Stärke solcher Carbon-Materialien, wie sie beispielsweise als Leichtbaumaterialien für Flugzeuge, Fahrräder, Boote und Autos bekannt sind, durch die spezielle Wahl von Ausrichtung und Schichtung der Kohlenstofffasern Kräfte in vorgegebenen Richtungen aufnehmen zu können und in anderen Richtungen flexibel, insbesondere elastisch flexibel zu sein. Typischerweise bestehen derartige Carbon-Materialien ganz überwiegend aus dem Kohlenstofffaser-Anteil. Die duroplastische Matrix dient im Wesentlichen lediglich der Fixierung der Kohlenstofffasern zueinander sowie als äußere Schutzbeschichtung. Diese besondere Designbarkeit von Carbon-Material macht sich die vorliegende Erfindung zu Nutze. Sie ermöglicht eine sehr dünne Ausgestaltung der Rückenschiene, die praktisch nicht aufträgt und gleichzeitig in Längserstreckungsrichtung, d.h. kranial-kaudal stauchfest ist. Zugleich bleibt aber eine sagittale Biegeelastizität erhalten, sodass sich der Benutzer sich nach wie vor nahezu ungehindert vorbeugen bzw. aufrichten kann. Diese sagittale Biegeelastizität sorgt zudem dafür, dass die Anlage der ventralen Flachseite der Rückenschiene an den der Wirbelsäule benachbarten Rückenmuskeln stets erhalten bleibt, sodass sich die Orthese hier abstützt. Dies bedeutet, dass in die Orthese eingeleitete Kräfte über die gesamte Orthesenlänge seitlich in besagte Rückenmuskulatur eingeleitet werden. Die Wirbelsäulenentlastung beruht daher auf einer Lastverlagerung von einer skelettalen Körperstruktur, nämlich von der Wirbelsäule, auf eine muskuläre Körperstruktur, nämlich auf besagte Rückenmuskeln. Muskuläre Strukturen sind zwar ermüdbar, aber deutlich schwerer schädigbar als skelettale Strukturen. So ist es stets Ziel jedes den Körper kräftigenden Trainings, die muskulären Strukturen derart aufzubauen, dass sie anstelle von skelettalen Strukturen äußere Kräfte aufzunehmen und zu dissipieren vermögen. Durch die erfindungsgemäße Orthese erfolgt die entsprechende Krafteinleitung in die muskulären Strukturen nunmehr direkt, d.h. unter Umgehung der skelettalen Strukturen, insbesondere der Wirbelsäule. Auf diese Weise kann die Kraft der muskulären Strukturen effizienter genutzt werden, wobei gleichzeitig die Krafteinwirkung auf die skelettalen Strukturen verringert wird.

Dabei ermöglichet es die erfindungsgemäße Materialwahl, die für die Kraftumleitung erforderlichen Stabilitäten, insbesondere in kranial-kaudaler Richtung, dauerhaft und gleichzeitig mit einer Flexibilität, insbesondere einer elastischen Flexibilität, in solchen Richtungen, in denen der Nutzer Bewegungsfreiheit benötigt, zu realisieren. Dies ist beim eingangs beschriebenen Stand der Technik, der entweder (im deaktivierten Zustand) eine völlige (nicht elastische) Flexibilität der Rückenschiene oder (im aktivierten Zustand) eine völlige Versteifung der Rückenschiene vorsah, nicht bzw. nur höchst unzureichend verwirklicht.

Im Ergebnis kann die erfindungsgemäße Orthese also ohne dem Benutzer lästig zu fallen dauerhaft getragen werden und erfüllt ihre Aufgabe ohne bewusste Umschaltung zwischen unterschiedlichen Zuständen, was die Orthese insbesondere auch im Kontext unvorhersehbar eintretender Belastungen, wie bspw. bei Rettungseinsätzen oder im Sport einsetzbar macht. Zudem ist das blattartige Carbon-Material so dünn und flach anliegend, dass es sich auch beim Aufliegen von Rückenlasten, bspw. Sauerstoffflaschen, nicht schmerzhaft in den Rücken des Benutzers drückt.

Bevorzugt weist die Rückenschiene zwei entlang ihrer Längserstreckungsrichtung parallel erstreckte, laterale Auflagebereiche sowie einen die Auflagebereiche verbindenden im Gebrauchsfall die Wirbelsäule des Nutzers überwölbenden Zentralbereich auf. Die Bedeutung der lateralen Auflagebereiche wurde oben bereits ausführlich erläutert. Durch die Überwölbung der Wirbelsäule mittels des Zentralbereichs werden gezielt die Dornfortsätze der Wirbelsäule entlastet, da ihr direkter Kontakt zur Rückenschiene vermieden wird. Besonders bevorzugt ist es dabei, wenn der Zentralbereich als eine sich gegenüber den Auflagebereichen nach dorsal wölbende Ausbuchtung ausgebildet ist. Mit anderen Worten bildet der Zentralbereich also nach ventral offenen Kanal, in welchen die Dornfortsätze berührungslos hineinragen können. Eine solche Formgebung erlaubt es, dass das Carbon-Material der Rückenschiene eine über deren Breite konstante Blattstärke aufweist. In der Regel genügt eine Blattstärke von weniger Millimetern, bspw. 1-3 mm, um die oben beschriebenen, vorteilhaften Eigenschaften der erfindungsgemäßen Orthese zu realisieren. Damit ist auch ein effektiver Schlagschutz für die empfindlichen Dornfortsätze gewährleistet, der sich insbesondere bei Stürzen bewähren kann.

Allerdings kann der im Zentralbereich realisierte Kanalähnlich einer Versteifungsrippe wirken, der die sagittale Biegeelastizität übermäßig einschränkt. Bei einer Weiterbildung der ist daher vorgesehen, dass der Zentralbereich mittels breitenzentrierter Querschlitze in eine Mehrzahl von über seine Länge verteilten Segmenten unterteilt ist. Dies ändert nichts an der Einstückigkeit der Rückenschiene und beeinträchtigt auch nicht die bevorzugte, vollflächige Anlage der Lateralbereiche an den Rückenstreckermuskeln. Die Flexibilität der Rückschiene in der medianen Sagittalebene wird jedoch erhöht. Durch spezielle Formgestaltung und Dimensionierung der Querschlitze können zudem auch die frontale Biegeelastizität sowie die Torsionselastizität beeinflusst und daher herstellerseitig auf das im Einzelfall gewünschte Maß eingestellt werden.

Zusätzlich zu ihrer sagittalen Biegeelastizität ist die Rückenschiene vorzugsweise auch frontal biegeelastische ausgestaltet, wobei der Betrag des frontalen Elastizitätsmoduls wenigstens eine Größenordnung größer als derjenige des sagittalen Elastizitätsmoduls ist. Damit erlaubt die erfindungsgemäße Orthese nicht nur ein Vorbeugen und Aufrichten des Nutzers, sondern - allerdings in beschränktem Maße - auch eine seitliche Verbiegung in einer Frontalebene. Derartige Bewegungen werden auch natürlicherweise von der Wirbelsäule nur in beschränktem Umfang zugelassen und können, im Übermaß angewendet, schnell zu einer ernsthaften Verletzung führen. Diese natürliche Abstufung der Beweglichkeiten wird von der erfindungsgemäßen Orthese in der beschriebenen Weiterbildung nachgeahmt. D. h. seitliche Biegungen werden zwar zugelassen, wobei der Nutzer allerdings daran "erinnert" wird, dass derartige Bewegungen günstigerweise auf ein Minimum reduziert werden sollten. Plötzlichen seitlichen Belastungen, denen der Nutzer in unvorhergesehener Weise ausgesetzt werden könnte, werden durch den hohen Elastizitätsmodul, d. h. die geringere Flexibilität in dieser Richtung abgefangen, sodass die Orthese zusätzlich einen Verletzungsschutz bietet.

In ähnlicher Weise ist bei einer Weiterbildung der Erfindung vorgesehen, dass die Rückenschiene um ihre Längserstreckungsrichtung elastisch tordierbar ausgebildet ist, wobei der Betrag ihres Torsions-Elastizitätsmoduls zwischen denjenigen ihres frontalen und ihres sagittalen Elastizitätsmoduls liegt. Auch dies entspricht einer Nachahmung der natürlichen Priorisierung unterschiedlicher Beweglichkeiten einer gesunden Wirbelsäule, die in der (medianen) Sagittalebene stark und leicht biegbar, etwas beschränkter und schwerer tordierbar und noch beschränkter und noch schwerer in der (medianen) Frontalebene biegbar ist.

Wesentliche vorteilhaften Eigenschaften der erfindungsgemäßen Orthese resultieren, wie beschrieben, aus der Gestaltung ihrer Rückenschiene. Diese kann allerdings nur erfindungsgemäß wirken, wenn sie im Gebrauchsfall korrekt positioniert ist. Grundsätzlich denkbar wäre es, eine separate Rückenschiene direkt am Rücken des Nutzers, bspw. durch Verklebung, zu fixieren. Dies würde jedoch die allgemeine Akzeptanz und Handhabbarkeit deutlich einschränken. Daher ist, wie eingangs bereits erwähnt, ein rucksackartiges Schulterträgersystem vorgesehen, mit welchem die Rückenschiene in Position gehalten wird. Erfindungsgemäß ist dabei jedes der beiden Trägerelemente des Schulterträgersystems aus einem blattartigen Carbon-Material, insbesondere dem gleichen Carbon-Material wie die Rückenschiene, aufgebaut und bildet im Gebrauchsfall einen die zugeordnete Schulter des Nutzers umgreifenden Ring, welcher, ausgehend vom kranialen Ende der Rückenschiene, flach an Schulter, Schlüsselbein und Brustmuskel des Nutzers anliegend parallel zu dessen Rippenbögen zur Rückenschiene zurückgeführt und im unteren Bereich deren oberen Drittels und/oder oberen Bereich ihres mittleren Drittels mit ihr verbunden ist. Bei dieser Ausführungsform wird also gerade kein Gurtsystem verwendet. Anstelle von Gurten, die (ggf. abgesehen von einer Zugfestigkeit) in sämtliche Richtungen flexibel sind, wird ein flach am Nutzer-Körper anliegender Carbon-Ring verwendet, der überall quer zu seiner jeweiligen Längserstreckungs- und Breitenrichtung biegeelastisch ist, in seiner Längserstreckungsrichtung jedoch stauch- bzw. zugfest und in seiner Breitenrichtung biegefest ist. Damit können die Trägerelemente eng anliegend an den Körperkonturen des Nutzers geführt werden und, ähnlich wie weiter oben im Kontext der lateralen Auflagebereiche der Rückenschiene erläutert, zur Einleitung von externen Kräften in muskuläre Strukturen, nämlich hier Schulter- und Brustmuskeln, dienen. Damit können auch diese Muskeln zur Aufnahme und Dissipation der externen Kräfte genutzt werden. Das führt zu einer weiteren Effizienzsteigerung der erfindungsgemäßen Wirbelsäulenentlastung.

Um bei einem solch selektiv steifen Schulterträgersystem eine gute Handhabbarkeit zu gewährleisten, ist bevorzugt vorgesehen, dass jedes Trägerelement zweiteilig aus einem oberen Halbringteil und einem unteren Halbringteil aufgebaut ist, wobei das obere Halbringteil materialeinheitlich einstückig mit der Rückenschiene ausgebildet ist und im Bereich seines freien Endes, welches im Gebrauchsfall im Bereich des Brustmuskels des Nutzers liegt, reversibel mit dem freien Ende des höhenverstellbar mit der Rückenschiene verbundenen, unteren Halbringteils verbindbar ist. Aufgrund der oben beschriebenen Biegeelastizität lassen sich bei einer solchen Gestaltung oberes und unteres Halbringteil im getrennten Zustand weit aufklappen, sodass ein bequemer Einstieg für den Nutzer möglich ist. Im Anschluss werden die freien Enden der Halbringteile im Brustmuskelbereich miteinander verbunden und der Trägerring geschlossen, sodass er dann das oben beschriebene selektiv steife Trägerelement bildet. Die materialeinheitlich einstückige Ausbildung der oberen Halbringteile mit der Rückenschiene ist im Hinblick auf die Vermeidung vieler Einzelteile und ggf. von Schwachstellen bildenden Verbindungsstellen günstig. Das untere Halbringteil sollte hingegen nur in Ausnahmenfällen einstückig mit der Rückenschiene verbunden sein. Allgemein wird es als günstiger angesehen, es reversibel, insbesondere höhenverstellbar mit der Rückenschiene zu verbinden, um eine Anpassbarkeit an unterschiedlich gewachsene Nutzer zu ermöglichen.

Als besonders praktikabel hat es sich erwiesen, wenn die freien Enden des oberen unteren Halbringteils im Gebrauchsfall einander überlappen und insbesondere mittels eines Klettverschlusses reversibel miteinander verbindbar sind. Auch das feste Ende des unteren Halbringteils kann mittels einer Klettverbindung mit der Rückenschiene verbunden sein, was insbesondere die oben erwähnte Höhenverstellbarkeit in technisch leicht umsetzbarer Weise realisiert. Die unteren Halbringteile der beiden Trägerelemente sind vorzugsweise miteinander einstückig ausgebildet. Sie gehen bevorzugt im Bereich ihrer Verbindung mit der Rückenschiene ineinander über.

Bei einer Weiterbildung der Erfindung ist vorgesehen, dass weiter eine radial biegeelastische, ventral offene, im Gebrauchsfall den Brustkorb des Nutzers flach anliegend von dorsal nach ventral umgreifende Rippenspange aus einem blattartigen Carbon-Material, vorzugsweise höhenverstellbar, insbesondere mittels einer Klettverbindung, mit der Rückenschiene verbunden ist. Die Rippenspange besteht vorzugswese aus dem gleichen blattartigen Carbon-Material wie die Rückenschiene. Aufgrund ihrer Biegeelastizität senkrecht zur Längserstreckungs- und Querrichtung legt sie sich automatisch an den Brustkorb des Nutzers an. Ihre Stauch- bzw. Zugfestigkeit in Längserstreckungsrichtung sowie ihre Biegefestigkeit in Breitenrichtung trägt zu einer stabilisierenden und zentrierenden Wirkung auf die Rückenschiene bei. Die vorzugsweise realisierte Höhenverstellbarkeit der Rippenspange dient der Anpassbarkeit an unterschiedliche Größen von Nutzern.

An den in Umfangsrichtung voneinander beabstandeten, freien Enden der Rippenspange sind bevorzugt Gurten fixiert, die zur Ausbildung einer den Abstand zwischen den freien Rippenspangen-Enden überspannenden Gurtverbindung reversibel miteinander verbindbar sind. Dies dient einer zusätzlichen Stabilität. Zudem ist es möglich, zwischen den freien Enden der Rippenspange, insbesondere auch in Verbindung mit den unteren Halbringteilen der Schultertrageelemente eine Brustplatte zu montieren, die dem Schutz des Solarplexus dienen kann.

Alternativ oder zusätzlich kann vorgesehen sein, dass weiter eine radial biegeelastische, ventral offene, im Gebrauchsfall den Körper des Nutzes entlang dessen Beckenkamms flach anliegend von dorsal nach ventral umgreifende Beckenspange aus einem blattartigen Carbon-Material, vorzugsweise höhenverstellbar, insbesondere mittels einer Klettverbindung, mit der Rückenschiene verbunden ist. Die Beckenspange besteht vorzugswese aus dem gleichen blattartigen Carbon-Material wie die Rückenschiene. Wie eingangs erläutert, besteht die zentrale Wirkung der Erfindung zwar nicht darin, externe Lasten auf das Becken des Nutzers abzuleiten; gleichwohl kann eine Ableitung von Restkräften, die noch nicht in der erfindungsgemäßen Weise auf muskuläre Strukturen abgeleitet werden konnten, auf den Beckenkamm sinnvoll sein. Dieser ist dann jedoch deutlich weniger belastet als bei Orthesen gemäß Stand der Technik.

Auch im Kontext der Beckenspange kann vorgesehen sein, dass an den in Umfangsrichtung voneinander beabstandeten, freien Enden der Beckenspange Gurte fixiert sind, die zur Ausbildung einer den Abstand zwischen den freien Beckenspangen-Enden überspannenden Gurtverbindung reversibel miteinander verbindbar sind. In Bezug auf die Wirkung und Vorteile dieser Maßnahme sei *mutatis mutandis* auf die obige Erläuterung zur Rippenspange verwiesen.

Weitere Einzelheiten und Vorteile der Erfindung ergeben sich aus der nachfolgenden, speziellen Beschreibung und den Zeichnungen.

### Kurzbeschreibung der Zeichnungen

Es zeigen:
- Figur 1:: eine perspektivische Darstellung des Grundgerüstes einer erfindungsgemäßen Thorax-Orthese,
- Figur 2:: eine Frontalansicht der Orthese von Figur 1,
- Figur 3:: eine Ansicht entlang der kranial-kaudalen Sichtlinie der Orthese von Figur 1 sowie
- Figur 4:: die Orthese von Figur 1 mit zusätzlicher Komfort- und Funktionsausstattung.

### Beschreibung bevorzugter Ausführungsformen

Gleiche Bezugszeichen in den Figuren deuten auf gleiche oder analoge Elemente hin.

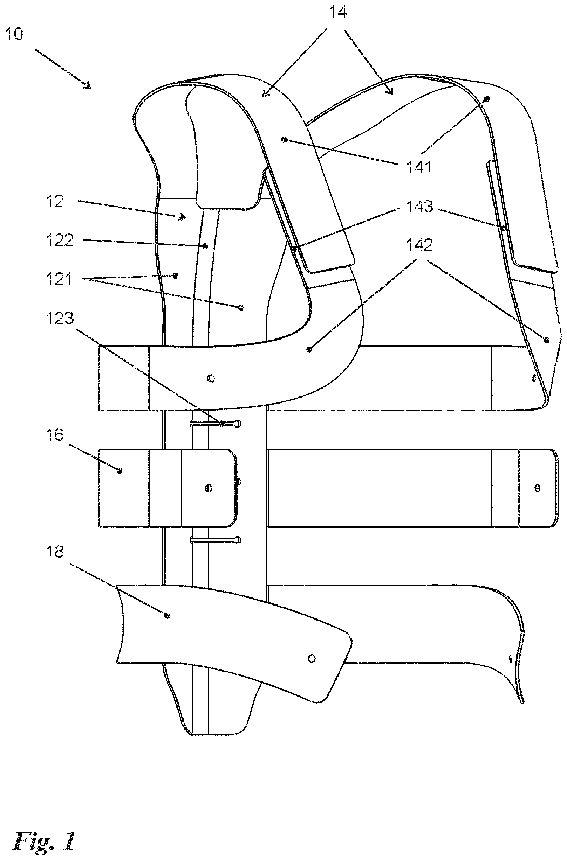

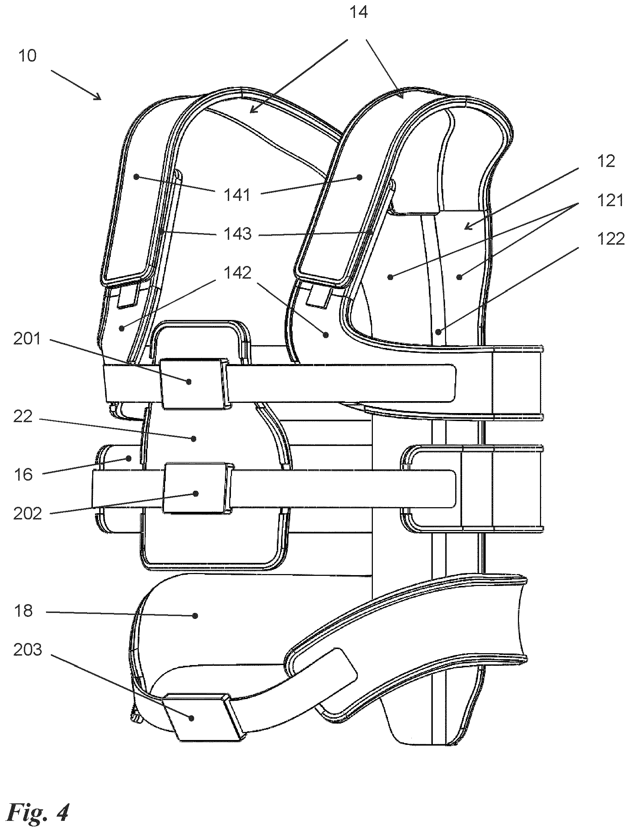

Die Figuren 1 bis 3 zeigen in unterschiedlichen Ansichten das Grundgerüst einer bevorzugten Ausführungsform einer erfindungsgemäßen Thorax-Orthese 10. Diese Figuren sollen zunächst gemeinsam diskutiert werden. Sie zeigen das im Wesentlichen aus blattartigem Carbon-Material gefertigte Grundgerüst der erfindungsgemäßen Orthese 10, das ggf., beispielsweise wie in Figur 4 illustriert, mit zusätzlichen Komfort- und Funktionselementen aufgerüstet werden kann.

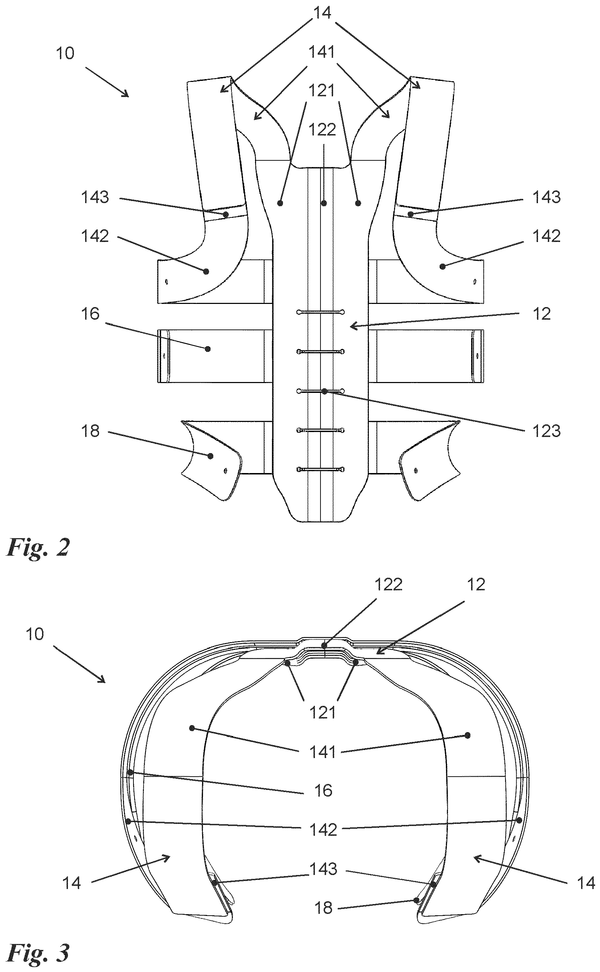

Wesentlicher Bestandteil der Orthese 10 ist deren Rückenschiene 12. Die Rückenschiene 12 besteht bei der dargestellten Ausführungsform aus zwei lateralen Auflagebereiche 121 und einem Zentralbereich 122, der als eine nach dorsal gerichtete Aufwölbung ausgebildet ist und einen kranial-kaudal verlaufenden, ventral offenen Kanal bildet. Im Gebrauchsfall ist die Rückenschiene 12 derart an den Rücken eines nicht dargestellten Benutzers angelegt, dass ihre lateralen Auflagebereiche 121 auf der der Wirbelsäule des Nutzers benachbarten Rückenstreckermuskulatur aufliegen und die Wirbelsäule selbst, insbesondere deren Dornfortsätze, berührungslos von dem Kanal des Zentralbereichs 122 überwölbt wird. Insbesondere in Figur 3 ist die Kanalstruktur des Zentralbereichs 122 gut erkennbar.

Über die Länge der Rückenschiene 12 verteilt sind mehrere Querschlitze 123 angeordnet, die den Kanal des Zentralbereichs 123 überspannen. Hierdurch wird der versteifenden Wirkung der Wölbung des Zentralbereichs 122 entgegengewirkt und die sagittale Biegeelastizität 12 vergrößert. Zudem bewirken die Querschlitze 123 eine Verbesserung der Ventilation. Bei der dargestellten Ausführungsform enden die Querschlitze 123 in seitlichen Rundlöchern. Hierdurch wird auch im Fall häufiger, starker Biegungen ein Ausreißen der Querschlitz-Enden vermieden. Zudem gewährleistet diese Formgebung gemeinsam mit einer je nach Einzelfall wählbaren Schlitzbreite eine herstellerseitig einstellbare Torsions- und Frontalelastizität, d.h. die Rückenschiene 12 ist frontal, d.h. in Figur 2 in der Zeichnungsebene, geringfügig biegbar sowie tordierbar, ohne dass die einander gegenüberliegenden Schlitzränder bei der Bewegung miteinander kollidieren und ggf. Kleidungs- oder gar Hautstücke einklemmen könnten.

Das kaudale Ende der Rückenschiene 12 ist zur Anpassung an die natürliche Anatomie im Lumbalbereich des Nutzers verjüngt, wobei auf eine gerundete und jegliche Ecken vermeidende Linienführung geachtet ist.

Am kranialen Enden der Rückenschiene 12 sind zwei obere Halbringteile 141 zweier Trägerelemente 14 angeformt. Sie bestehen aus dem gleichen blattartigen Carbon-Material wie die Rückenschiene 12. Sie verlaufen im Gebrauchsfall über die Schultern des Nutzers und enden mit ihren freien Enden etwa im Bereich seiner Brustmuskulatur. Hier sind sie reversibel, insbesondere mittels eines Klettverschlusses 143 mit den freien Enden unterer Halbringteile 142 verbunden, die sich vom Brustmuskelbereich parallel zu den Rippenbögen des Nutzers von ventral nach dorsal zur Rückenschiene 12 erstrecken. Dort sind sie vorzugsweise reversibel, insbesondere mittels eines weiteren Klettverschlusses an der dorsalen Außenseite der Rückenschiene 12 fixiert. Bei der dargestellten, bevorzugten Ausführungsform sind die beiden unteren Halbringteile 142 einstückig miteinander ausgebildet und gehen insbesondere im Bereich ihrer Fixierung an der Rückenschiene 12 materialeinheitlich ineinander über. Die Reversibilität ihrer Fixierung an der Rückenschiene 12 erlaubt eine Höhenverstellung und damit einer Größenverstellung der Armausschnitte der Trägerelemente 14, sodass die Orthese 12 in gewissen Grenzen an unterschiedlich groß gewachsene Nutzer angepasst werden kann. Es ist daher möglich, die Fertigung auf eine geringe Anzahl von Grundgrößen zu beschränken und durch eine nutzerseitige Feinabstimmung eine individuell optimierte Anpassung zu ermöglichen. Aufgrund der im allgemeinen Teil der Beschreibung ausführlich diskutierten Materialeigenschaften blattartigen Carbon-Materials sind die oberen Halbringteile bei geöffnetem Klettverschluss 143 ohne großen Kraftaufwand elastisch nach oben aufbiegbar. Die unteren Halbringteile 142 hingegen sind nach seitlich außen aufbiegbar. Dies ermöglicht dem Nutzer einen leichten Einstieg in die Orthese 12.

Nach Fixierung der freien Enden von oberen und unteren Halbringteilen 141, 142 mittels der Klettverschlüsse 143 unterbinden die Halbringteile 141, 142 jedoch den Biegefreiheitsgrad des jeweils anderen Halbringelementes 142, 141, sodass insgesamt ein im Wesentlichen steifer Trägerring entsteht, der sich unmittelbar an der Schulter- und Brustmuskulatur des Nutzers abstützt. Gemeinsam mit der Rückenschiene 12 sorgen die Trägerelemente 14 daher für eine Ableitung von externen Kräften weg von der Wirbelsäule des Nutzers hin zu einer breiten Palette muskulärer Strukturen. Diese können die Kräfte gemeinsam aufnehmen, ohne dass einzelne Muskeln überlastet würden. Dies führt zu einer besonderen Effizienz der Wirbelsäulenentlastung durch die erfindungsgemäße Orthese und damit zur deutlichen Steigerung der Leistungsfähigkeit des Nutzers.

Bei der dargestellten Ausführungsform ist kaudal der unteren Halbringteile 142 der Trägerelemente 14 eine Rippenspange 16 angeordnet, die, ähnlich wie die unteren Halbringelemente 142, vorzugsweise reversibel, insbesondere mittels eines Klettverschlusses an der dorsalen Außenseite der Rückenschiene 12 fixiert sind. Auch sie sind bevorzugt aus dem gleichen Carbon-Material wie Rückenschiene 12 und Trägerelemente 14 gefertigt und schmiegen sich biegeelastisch im Bereich der unteren Rippenbögen an den Oberkörper des Nutzers an. Zum Einstieg sind sie nach seitlich außen aufbiegbar. Sie dienen primär der zentrierenden Fixierung der Rückenschiene 12 in deren Mittelbereich.

Nahe des kaudalen Endes der Rückenschiene 12 ist bei der gezeigten Ausführungsform zudem eine Beckenspange 18 vorgesehen, die, vorzugsweise ebenfalls reversibel, insbesondere ebenfalls mittels eines Klettverschlusses, an der dorsalen Außenseite der Rückenschiene 12 fixiert ist. Die Beckenspange 18 umgreift den Körper des Nutzers von dorsal nach ventral entlang seines Beckenkamms. Anders als bei der Rippenspange 16 erlaubt ihre in den Figuren 1 und 2 besonders gut erkennbare, leicht gekippte Biegung eine vertikale Lastabstützung auf dem Beckenkamm. Dies ist jedoch, wie im allgemeinen Teil der Beschreibung ausführlich erläutert, lediglich ein Sekundäreffekt. Primär dient die Beckenspange 18 einer zentrierenden Fixierung der Rückenschiene 12 in deren kaudalem Endbereich.

Das reine Carbon-Gerüst (ggf. mit Verbindungsvorrichtungen) insbesondere Klettverschlüssen, wie in den Figuren 1 bis 3 gezeigt, genügt grundsätzlich zur Erzielung der im allgemeinen Teil der Beschreibung erläuterten, erfindungsgemäßen Wirkungen. Es ist jedoch ohne weiteres möglich, beispielsweise aus Komfortgründen, eine zusätzliche Ausstattung vorzunehmen. So kann das Carbon-Gerüst mit Polsterelementen, beispielsweise Gel-Polster oder aus Neoprenschichten, ganz oder lokal verkleidet oder beschichtet sein. Eine entsprechend aufgerüstete Orthese 12 ist in Figur 4 dargestellt.

Außer besagten Polsterungen weist die Orthese 12 von Figur 4 zusätzliche Gurtverbindungen 201, 202, 203 auf, die die einander gegenüberliegenden Bereiche bzw. Enden der unteren Halbringteile 142 (Gurtverbindung 201), Rippenspange 16 (Gurtverbindung 202) bzw. Beckenspange (Gurtverbindung 203) miteinander verbinden. Hierdurch kann eine stabilere Fixierung der Rückenschiene 12 erreicht werden, die durch die Gurtverbindungen 201, 202, 203 insbesondere stärker an die Wirbelsäule des Nutzers herangezogen werden kann.

Bei der dargestellten Ausführungsform ist im Bereich der Verschlüsse der beiden oberen Gurtverbindungen 201, 202 eine Brustplatte 22 fixiert. Diese ist bevorzugt ebenfalls aus einem blattartigen Carbon-Material, insbesondere dem gleichen Carbon-Material wie die Rückenschiene 12, gefertigt und kann auf ihrer Innenseite mit einer Polsterung versehen, zum Beispiel beschichtet sein.

Eine solche Brustplatte 22 bietet einen effektiven Schlagschutz für den Solarplexus des Nutzers. Zudem gebietet sie eine weitere Kontaktfläche zu muskulären Strukturen des Nutzers, die zur Aufnahme der über die Orthese 12 abgeleitet, externen Kräfte genutzt werden kann.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben.

### Bezugszeichenliste

- 10: Orthese
- 12: Rückenschiene
- 121: lateraler Auflagebereich von 12
- 122: Zentralbereich von 12
- 123: Querschlitz
- 14: Trägerelement
- 141: oberes Halbringteil von 14
- 142: unteres Halbringteil von 14
- 143: Klettverschluss
- 16: Rippenspange
- 18: Beckenspange
- 201: Gurtverbindung
- 202: Gurtverbindung
- 203: Gurtverbindung
- 22: Brustplatte

## Patentansprüche

1. Thorax-Orthese (10) zur bedarfsweisen Entlastung der Wirbelsäule eines menschlichen Nutzers,
umfassend
- eine einstückig aus einem blattartigen, in ihrer Längserstreckungsrichtung stauchfesten Carbon-Material aufgebaute Rückenschiene (12), die im Gebrauchsfall mit einer ventralen Flachseite im Bereich der Brust- und zumindest der oberen Lendenwirbel des Nutzers an den der Wirbelsäule des Nutzers beidseitig benachbarten Rückenstreckermuskeln anliegt, sowie
- ein mit der Rückenschiene (12) verbundenes Schulterträgersystem mit zwei im Gebrauchsfall die Schultern des Nutzers rucksackartig umgreifenden Trägerelementen (14),
**dadurch gekennzeichnet,**
**dass** das Carbon-Material der Rückenschiene sagittal biegeelastisch ist und jedes Trägerelement (14) des Schulterträgersystems aus einem blattartigen Carbon-Material aufgebaut ist und im Gebrauchsfall einen die zugeordnete Schulter des Nutzers umgreifenden Ring bildet, welcher, ausgehend vom kranialen Ende der Rückenschiene (12), flach an Schulter, Schlüsselbein und Brustmuskel des Nutzer anliegend parallel zu dessen Rippenbögen zur Rückenschiene (12) zurückgeführt und im unteren Bereich deren oberen Drittels und/oder oberen Bereich ihres mittleren Drittels mit ihr verbunden ist.

2. Thorax-Orthese (10) nach Anspruch 1,
**dadurch gekennzeichnet,**
**dass** das Trägerelement (14) zweiteilig aus einem oberen Halbringteil (141) und einem unteren Halbringteil (142) aufgebaut ist, wobei das obere Halbringteil (141) materialeinheitlich einstückig mit der Rückenschiene (12) ausgebildet ist und im Bereich seines freien Endes, welches im Gebrauchsfall im Bereich des Brustmuskels des Nutzers liegt, reversibel mit dem freien Ende des höhenverstellbar mit der Rückenschiene (12) verbundenen, unteren Halbringteils (142) verbindbar ist.

3. Thorax-Orthese (10) nach Anspruch 2,
**dadurch gekennzeichnet,**
**dass** die freien Enden des oberen Halbringteils (141) und des unteren Halbringteils (142) im Gebrauchsfall einander überlappen und reversibel, insbesondere mittels eines Klettverschlusses (143), miteinander verbindbar sind.

4. Thorax-Orthese (10) nach einem der Ansprüche 2 bis 3,
**dadurch gekennzeichnet,**
**dass** das untere Halbringteil (142) mittels einer Klettverbindung mit der Rückenschiene (12) verbunden ist.

5. Thorax-Orthese (10) nach einem der vorangehenden Ansprüche,
**dadurch gekennzeichnet,**
**dass** die Rückenschiene (12) zwei entlang ihrer Längserstreckungsrichtung parallel erstreckte, laterale Auflagebereiche (121) sowie einen die Auflagebereiche (121) verbindenden, im Gebrauchsfall die Wirbelsäule des Nutzers überwölbenden Zentralbereich (122) aufweist.

6. Thorax-Orthese (10) nach Anspruch 5,
**dadurch gekennzeichnet,**
**dass** der Zentralbereich (122) als eine sich gegenüber den Auflagebereichen nach dorsal wölbende Ausbuchtung ausgebildet ist.

7. Thorax-Orthese (10) nach Anspruch 6,
**dadurch gekennzeichnet,**
**dass** das Carbon-Material der Rückenschiene (12) eine über deren Breite konstante Blattstärke aufweist.

8. Thorax-Orthese (10) nach einem der Ansprüche 5 bis 7,
**dadurch gekennzeichnet,**
**dass** der Zentralbereich (122) mittels breitenzentrierter Querschlitze (123) in eine Mehrzahl von über seine Länge verteilten Segmenten unterteilt ist.

9. Thorax-Orthese (10) nach einem der vorangehenden Ansprüche,
**dadurch gekennzeichnet,**
**dass** die Rückenschiene (12) frontal biegeelastisch ausgebildet ist, wobei der Betrag des frontalen Elastizitätsmoduls wenigstens eine Größenordnung größer als derjenige des sagittalen Elastizitätsmoduls ist.

10. Thorax-Orthese (10) nach Anspruch 9,
**dadurch gekennzeichnet,**
**dass** die Rückenschiene (12) um ihre Längserstreckungsrichtung elastisch tordierbar ausgebildet ist, wobei der Betrag ihres Torsions-Elastizitätsmoduls zwischen denjenigen ihres frontalen und ihres sagittalen Elastizitätsmoduls liegt.

11. Thorax-Orthese (10) nach einem der vorangehenden Ansprüche,
**dadurch gekennzeichnet,**
**dass** weiter eine radial biegeelastische, ventral offene, im Gebrauchsfall den Brustkorb des Nutzers flach anliegend von dorsal nach ventral umgreifende Rippenspange (16) aus einem blattartigen Carbon-Material mit der Rückenschiene (12) verbunden ist.

12. Thorax-Orthese (10) nach Anspruch 11,
**dadurch gekennzeichnet,**
**dass** an den in Umfangsrichtung voneinander beabstandeten, freien Enden der Rippenspange (12) Gurte fixiert sind, die zur Ausbildung einer den Abstand zwischen den freien Rippenspangen-Enden überspannenden Gurtverbindung (202) reversibel miteinander verbindbar sind.

13. Thorax-Orthese (10) nach einem der vorangehenden Ansprüche,
**dadurch gekennzeichnet,**
**dass** weiter eine radial biegeelastische, ventral offene, im Gebrauchsfall den Körper des Nutzers entlang dessen Beckenkamms flach anliegend von dorsal nach ventral umgreifende Beckenspange (18) aus einem blattartigen Carbon-Material mit der Rückenschiene (12) verbunden ist.

14. Thorax-Orthese (10) nach Anspruch 13,
**dadurch gekennzeichnet,**
**dass** an den in Umfangsrichtung voneinander beabstandeten, freien Enden der Beckenspange (18) Gurte fixiert sind, die zur Ausbildung einer den Abstand zwischen den freien Beckenspangen-Enden überspannenden Gurtverbindung (203) reversibel miteinander verbindbar sind.

## Claims

1. Thoracic orthosis (10) for relieving a human user's spine when required, comprising
- a back rail (12) made in one piece from a sheet-like carbon material which is compression-resistant in its direction of longitudinal extension and resting, when in use, with a ventral flat side against back extensor muscles adjacent to both sides of the user's spine in the region of the thoracic and at least the upper lumbar vertebrae of the user, and
- a shoulder carrier system connected to the back rail (12) and having two carrier elements (14) reaching around the user's shoulders in a backpack-style when in use,
**characterized in**
**that** the carbon material of the back rail is sagittally bending-elastic and each carrier element (14) of the shoulder carrier system is made from a sheet-like carbon material and, when in use, forms a ring embracing the respectively associated shoulder of the user and, starting from the cranial end of the back rail (12), being guided back parallel to the user's rib arches to the back rail (12), to which it is connected in the lower region of the back rail's (12) upper third and/or the upper region of the back rail's (12) middle third, lying flat against the user's shoulder, collarbone and chest muscle.

2. Thoracic orthosis (10) according to claim 1,
**characterized in**
**that** the carrier element (14) is constructed in two parts, namely an upper half-ring part (141) and a lower half-ring part (142), the upper half-ring part (141) being formed integrally with the back rail (12) as a single piece of material and being reversibly connectable in the region of its free end, which, when in use lies in the region of the user's chest muscle, to the free end of the lower half-ring part (142), which is connected to the back rail (12) in a height-adjustable manner.

3. Thoracic orthosis (10) according to claim 2,
**characterized in**
**that** the free ends of the upper half-ring part (141) and of the lower half-ring part (142) overlap one another when in use and can be connected to one another reversibly, in particular by means of a hook-and-loop fastener (143).

4. Thoracic orthosis (10) according to one of claims 2 to 3,
**characterized in**
**that** the lower half-ring part (142) is connected to the back rail (12) by means of a hook-and-loop connection.

5. Thoracic orthosis (10) according to one of the preceding claims
**characterized in**
**that** the back rail (12) has two lateral support regions (121) extending parallel along the longitudinal direction of extension of the back rail (12) and a central region (122) connecting the support regions (121) and arching over the user's spine when in use.

6. Thoracic orthosis (10) according to claim 5,
**characterized in**
**that** the central region (122) is shaped as a bulge which curves dorsally with respect to the support regions.

7. Thoracic orthosis (10) according to claim 6,
**characterized in**
**that** the carbon material of the back rail (12) has a constant sheet thickness across its width.

8. Thoracic orthosis (10) according to any one of claims 5 to 7,
**characterized in**
**that** the central region (122) is divided by means of width-centered transverse slits (123) into a plurality of segments distributed across its length).

9. Thoracic orthosis (10) according to one of the preceding claims,
**characterized in**
**that** the back rail (12) is designed to be frontally bending-elastic, the amount of its frontal modulus of elasticity being at least one order of magnitude greater than that of its sagittal modulus of elasticity.

10. Thoracic orthosis (10) according to claim 9,
**characterized in**
**that** the back rail (12) is designed to be torsionally elastic about its direction of longitudinal extension, the amount of its torsional modulus of elasticity being between those of its frontal and its sagittal modulus of elasticity.

11. Thoracic orthosis (10) according to one of the preceding claims,
**characterized in**
**that**, furthermore, a radially bending-elastic rib bracket (16) being open ventrally and, when in use, embracing the user's thorax flatly from dorsal to ventral, and being made of a sheet-like carbon material is connected to the back rail (12).

12. Thoracic orthosis (10) according to claim 11,
**characterized in**
**that** straps are fixed to the free ends of the rib bracket (16), said free ends being spaced apart from one another in the circumferential direction and said straps being reversibly connectable to one another to form a strap connection (202) spanning the distance between the free ends of the rib bracket (16).

13. Thoracic orthosis (10) according to one of the preceding claims,
**characterized in**
**that**, furthermore, a radially bending-elastic pelvic bracket (18) is connected to the back rail (12), said pelvic bracket (18) being ventrally open, made of a sheet-like carbon material, and embracing, when in use, the user's body flatly from dorsal to ventral along the user's iliac crest.

14. Thoracic orthosis (10) according to claim 13,
**characterized in**
**that** straps are fixed to the free ends of the pelvic bracket (18), said free ends being spaced apart from one another in the circumferential direction and said straps being reversibly connectable to one another to form a strap connection (203) spanning the distance between the free ends of the pelvic bracket (18).

## Revendications

1. Orthèse thoracique (10) pour soulager la colonne vertébrale d'un utilisateur humain en cas de besoin,
comprenant
- un rail dorsal (12) construit d'une seule pièce à partir d'un matériau en carbone en forme de feuille, résistant à l'écrasement dans sa direction d'extension longitudinale, qui, en cas d'utilisation, s'applique par un côté plat ventral dans la zone des vertèbres thoraciques et au moins des vertèbres lombaires supérieures de l'utilisateur contre les muscles extenseurs dorsaux voisins des deux côtés de la colonne vertébrale de l'utilisateur, ainsi que
- un système de support d'épaule relié au rail dorsal (12) et comportant deux éléments de support (14) qui, en cas d'utilisation, entourent les épaules de l'utilisateur à la manière d'un sac à dos,
**caractérisé en ce**
**que** le matériau en carbone du rail dorsal est élastique en flexion dans le sens sagittal et
chaque élément de support (14) du système de support d'épaule est constitué d'un matériau en carbone en forme de feuille et forme, en cas d'utilisation, un anneau entourant l'épaule associée de l'utilisateur, lequel, en partant de l'extrémité crânienne du rail dorsal (12), revient au rail dorsal (12) en s'appliquant à plat contre l'épaule, la clavicule et le muscle pectoral de l'utilisateur, parallèlement à ses arcs costaux, et est relié à celui-ci dans la zone inférieure de son tiers supérieur et/ou dans la zone supérieure de son tiers médian.

2. Orthèse thoracique (10) selon la revendication 1,
**caractérisé en ce**
**que** l'élément de support (14) est constitué de deux parties, à savoir d'une partie supérieure de demi-anneau (141) et d'une partie inférieure de demi-anneau (142), la partie supérieure de demi-anneau (141) étant réalisée d'une seule pièce du même matériau avec le rail dorsal (12) et pouvant être reliée de manière réversible, dans la zone de son extrémité libre qui, en cas d'utilisation, se trouve dans la zone du muscle pectoral de l'utilisateur, à l'extrémité libre de la partie inférieure de demi-anneau (142) reliée de manière réglable en hauteur au rail dorsal (12).

3. Orthèse thoracique (10) selon la revendication 2,
**caractérisé en ce**
**que** les extrémités libres de la partie supérieure de demi-anneau (141) et de la partie inférieure de demi-anneau (142) se chevauchent en cas d'utilisation et peuvent être reliées l'une à l'autre de manière réversible, en particulier au moyen d'une fermeture velcro (143).

4. Orthèse thoracique (10) selon l'une des revendications 2 à 3,
**caractérisé en ce**
**que** la partie inférieure de demi-anneau (142) est reliée au rail dorsal (12) au moyen d'une liaison velcro.

5. Orthèse thoracique (10) selon l'une des revendications précédentes
**caractérisé en ce**
**que** le rail dorsal (12) présente deux zones d'appui latérales (121) s'étendant parallèlement le long de sa direction d'extension longitudinale, ainsi qu'une zone centrale (122) reliant les zones d'appui (121) et surplombant la colonne vertébrale de l'utilisateur en cas d'utilisation.

6. Orthèse thoracique (10) selon la revendication 5,
**caractérisé en ce**
**que** la zone centrale (122) est réalisée sous la forme d'un renflement se courbant en direction dorsale par rapport aux zones d'appui.

7. Orthèse thoracique (10) selon la revendication 6,
**caractérisé en ce**
**que** le matériau en carbone du rail dorsal (12) présente une épaisseur de feuille constante sur sa largeur.

8. Orthèse thoracique (10) selon l'une des revendications 5 à 7,
**caractérisé en ce**
**que** la zone centrale (122) est divisée en une pluralité de segments répartis sur sa longueur au moyen de fentes transversales (123) centrées sur la largeur.

9. Orthèse thoracique (10) selon l'une des revendications précédentes,
**caractérisé en ce**
**que** le rail dorsal (12) est réalisé de manière d'être élastique en flexion dans le plan frontal, la valeur du module d'élasticité frontal étant supérieure d'au moins un ordre de grandeur à celle du module d'élasticité sagittal.

10. Orthèse thoracique (10) selon la revendication 9,
**caractérisé en ce**
**que** le rail dorsal (12) est réalisé de manière à pouvoir être tordue élastiquement autour de sa direction d'extension longitudinale, la valeur de son module d'élasticité en torsion étant comprise entre celles de son module d'élasticité frontal et de son module d'élasticité sagittal.

11. Orthèse thoracique (10) selon l'une des revendications précédentes,
**caractérisé en ce**
**qu'**en outre, une barrette costale (16) d'un matériau de carbone en forme de feuille, élastique en flexion radiale, ouverte sur le côté ventral et entourant à plat, en cas d'utilisation, la cage thoracique de l'utilisateur, du côté dorsal vers le côté ventral, est reliée au rail dorsal (12).

12. Orthèse thoracique (10) selon la revendication 11,
**caractérisé en ce**
**que** des sangles sont fixées aux extrémités libres de la barrette de côtes (12), espacées les unes des autres dans le sens circonférentiel, qui peuvent être reliées entre elles de manière réversible pour former une liaison par sangle (202) couvrant la distance entre les extrémités libres de la barrette de côtes (16).

13. Orthèse thoracique (10) selon l'une des revendications précédentes,
**caractérisé en ce**
**qu'**il est en outre relié au rail dorsal (12) une barrette pelvienne (18) en un matériau en carbone en forme de feuille, radialement élastique en flexion, ouverte sur le côté ventral, qui, en cas d'utilisation, entoure le corps de l'utilisateur en s'appliquant à plat le long de sa crête iliaque, du côté dorsal vers le côté ventral.

14. Orthèse thoracique (10) selon la revendication 13,
**caractérisé en ce**
**que** des sangles sont fixées aux extrémités libres de la barrette pelvienne (18), espacées les unes des autres dans le sens circonférentiel, qui peuvent être reliées entre elles de manière réversible pour former une liaison par sangle (203) couvrant la distance entre les extrémités libres de la barrette pelvienne (18).
